(19) Europäisches Patentamt
European Patent Office
Office européen des brevets

(11) Veröffentlichungsnummer: **0 065 204**
**B1**

(12) # EUROPÄISCHE PATENTSCHRIFT

(45) Veröffentlichungstag der Patentschrift: **15.01.86**

(51) Int. Cl.⁴: **C 07 D 249/08**, C 07 D 233/60, A 61 K 31/41, A 61 K 31/415

(21) Anmeldenummer: **82103851.0**

(22) Anmeldetag: **05.05.82**

(54) **3-Substituierte 1-Azolyl-3-methyl-1-phenoxy-butan-2-one und -ole, Verfahren zu ihrer Herstellung und ihre Verwendung als Fungizide.**

(30) Priorität: **15.05.81 DE 3119390**

(43) Veröffentlichungstag der Anmeldung: **24.11.82 Patentblatt 82/47**

(45) Bekanntmachung des Hinweises auf die Patenterteilung: **15.01.86 Patentblatt 86/3**

(84) Benannte Vertragsstaaten: **AT BE CH DE FR GB IT LI NL**

(56) Entgegenhaltungen:
**EP-A-0 049 111**
**FR-A-2 360 309**
**US-A-4 215 127**

(73) Patentinhaber: **BAYER AG, Konzernverwaltung RP Patentabteilung, D-5090 Leverkusen 1 Bayerwerk (DE)**

(72) Erfinder: **Kraatz, Udo, Dr., Körner Strasse 6, D-5090 Leverkusen 1 (DE)**
Erfinder: **Klauke, Erich, Dr., Eichendorffweg 8, D-5068 Odenthal (DE)**
Erfinder: **Jäger, Gerhard, Dr., Gellertstrasse 18, D-5090 Leverkusen 1 (DE)**
Erfinder: **Büchel, Karl Heinz, Dr. Prof., Dabringhausener Strasse 42, D-5093 Burscheid (DE)**
Erfinder: **Frohberger, Paul- Ernst, Dr., Willi-Baumeister- Strasse 5, D-5090 Leverkusen 1 (DE)**
Erfinder: **Brandes, Wilhelm, Dr., Eichendorffstrasse 3, D-5653 Leichlingen 1 (DE)**

**EP 0 065 204 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue 3-substituiert 1-Azolyl-3-methyl-1-phenoxy-butan-2-one und -ole, ein Verfahren zu ihrer Herstellung und ihre Verwendung als Fungizide.

Es ist bereits bekannt geworden, daß 1-Azolyl-3,3-di-methyl-1-phenoxy-butan-2-one und -ole allgemein gute fungizide Eigenschaften aufweisen (vergleiche DE-PS 22 01 063, DE-OS 23 24 010 und DE-OS 23 25 156. Die Wirkung dieser Verbindungen ist jedoch, insbesondere bei niedrigen Aufwandmengen und -konzentrationen, nicht immer ganz befriedigend; dasselbe gilt für aus der US-PS 4 215 127 bekannt gewordene Verbindungen.

Es wurden neue 3-substituierte 1-Azolyl-3-methyl-1-phenoxy-butan-2-one und -ole der allgemeinen Formel

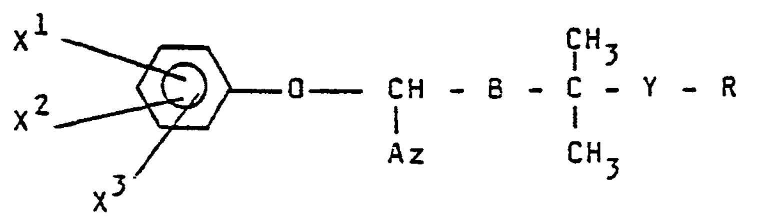 (I)

in welcher

Az für 1, 2, 4-Triazol-1-yl, 1, 2, 4-Triazol-4-yl oder Imidazol-1-yl steht,

B für die Ketogruppe oder die CH(OH)-Gruppe steht,

Y für Sauerstoff oder Schwefel steht,

R für Halogenalkyl mit 1 bis 4 Kohlenstoffatomen und 1 bis 5 gleichen oder verschiedenen Halogenatomen, sowie für gegebenenfalls substituiertes Phenyl und Benzyl steht, wobei als Phenylsubstituenten jeweils Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoffatomen und 1 bis 5 gleichen oder verschiedenen Halogenatomen, Nitro, Cyano, Alkoxycarbonyl mit 1 bis 4 Kohlenstoffatomen im Alkylteil, sowie gegebenenfalls durch Halogen substituiertes Phenyl zu nennen sind; sowie auch für geradkettiges oder verzweigtes Alkyl mit 1 bis 6 Kohlenstoffatomen steht, wenn Y für Schwefel steht;

$X^1$ für Wasserstoff, Halogen, für Alkyl, Alkoxy, Alkylthio und Alkylsulfonyl mit jeweils 1 bis 4 Kohlenstoffatomen, für Halogenalkyl mit 1 bis 4 Kohlenstoffatomen und 1 bis 5 gleichen oder verschiedenen Halogenatomen, für Nitro, Cyano, Alkoxycarbonyl mit 1 bis 4 Kohlenstoffatomen im Alkylteil, sowie für gegebenenfalls durch Halogen substituiertes Phenyl steht;

$X^2$ für Wasserstoff, Halogen, für Alkyl, Alkoxy, Alkylthio und Alkylsulfonyl mit jeweils 1 bis 4 Kohlenstoffatomen, für Halogenalkyl mit 1 bis 4 Kohlenstoffatomen und 1 bis 5 gleichen oder verschiedenen Halogenatomen, für Nitro, Cyano, Alkoxycarbonyl mit 1 bis 4 Kohlenstoffatomen im Alkylteil, sowie für gegebenenfalls durch Halogen substituiertes Phenyl steht,

$X^3$ für Wasserstoff, Halogen, für Alkyl, Alkoxy und Alkylthio mit jeweils 1 bis 4 Kohlenstoffatomen, sowie für Halogenalkyl mit 1 bis 4 Kohlenstoffatomen und 1 bis 5 gleichen oder verschiedenen Halogenatomen steht

sowie deren physiologisch verträglichen Säureadditions-Salze und Metallsalz-Komplexe gefunden.

Diejenigen Verbindungen der Formel (I), in welchen B für die CH(OH)-Gruppe steht, besitzen zwei asymmetrische Kohlenstoffatome; sie können deshalb in zwei geometrischen Isomeren (threo- und erythro-Form) vorliegen, die in unterschiedlichen Mengenverhältnissen anfallen können. In beiden Fällen liegen sie als optische Isomere vor.

Weiterhin wurde gefunden, daß man die 3-substituierten 1-Azolyl-3-methyl-1-phenoxy-butan-2-one und -ole der Formel (I) erhält, wenn man Halogenetherketone der Formel

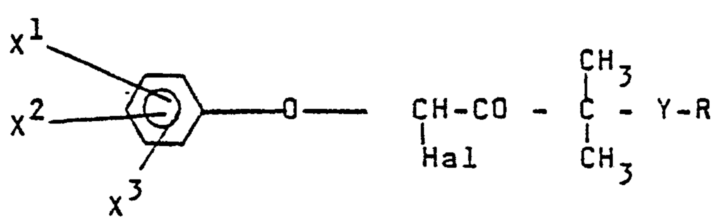 (II)

in welcher Hal für Halogen, vorzugsweise Chlor oder Brom steht und

R, $X^1$, $X^2$, $X^3$ und Y die oben angegebene Bedeutung haben,

mit 1, 2, 4-Triazol oder Imidazol in Gegenwart eines Verdünnungsmittels und in Gegenwart eines Säurebindemittels umsetzt, und gegebenenfalls noch die so erhaltenen Keto-Derivate der Formel

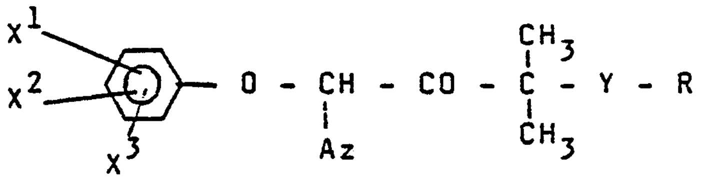

(Ia)

in welcher
Az, R, $X^1$, $X^2$,
$X^3$ und Y die oben angegebene Bedeutung haben,
nach bekannten Methoden in üblicher Weise reduziert.

An die so erhaltenen Verbindungen der Formel (I) können gegebenenfalls anschließend eine Säure oder ein Metallsalz addiert werden. In manchen Fällen erweist es sich als vorteilhaft, die Verbindungen der Formel (I) über ihre Salze in reiner Form zu erhalten.

Die neuen 3-substituierten 1-Azolyl-3-methyl-1-phenoxy-butan-2-one und -ole der Formel (I) weisen starke fungizide Eigenschaften auf. Dabei zeigen überraschenderweise die erfindungsgemäßen Verbindungen eine bessere fungizide Wirkung als die aus dem Stand der Technik bekannten 1-Azolyl-3, 3-dimethyl-1-phenoxy-butan-2-one und -ole, die chemisch und wirkungsmäßig naheliegende Verbindungen sind.

Außerdem sind die neuen 3-substituierten 1-Azolyl-3-methyl-1-phenoxy-butan-2-one und -ole der Formel (I) interessante Zwischenprodukte zur Herstellung von weiteren Pflanzenschutz-Wirkstoffen. So können durch entsprechende Umsetzung funktionelle Derivate der Ketogruppe erhalten werden, wie z.B. Oxime und Oximether, Hydrazone und Ketale. Außerdem können die Verbindungen der Formel (I) an der Hydroxy-Gruppe in üblicher Weise in die entsprechenden Ether überführt werden, bzw. können durch Umsetzung mit z.B. Acylhalogeniden oder Carbamoylchloriden in prinzipiell bekannter Weise Acyl- oder Carbamoyl-Derivate erhalten werden.

Die erfindungsgemäßen Stoffe stellen somit eine wesentliche Bereicherung der Technik dar.

Die erfindungsgemäßen 3-substituierten 1-Azolyl-3-methyl-1-phenoxy-butan-2-one und -ole sind durch die Formel (I) allgemein definiert.

Bevorzugt sind diejenigen Verbindungen der Formel (I), in denen

R für Halogenalkyl mit 1 bis 2 Kohlenstoffatomen und 1 bis 5 gleichen oder verschiedenen Halogenatomen, wie insbesondere Fluor und Chlor, sowie für gegebenenfalls substituiertes Phenyl oder Benzyl steht, wobei als Phenylsubstituenten jeweils genannt seien: Fluor, Chlor, Methyl, Ethyl, Isopropyl, tert.-Butyl, Methoxy, Methylthio, Trifluormethyl, Trifluormethoxy, Trifluormethylthio, Nitro, Cyano, sowie gegebenenfalls durch Fluor und Chlor substituiertes Phenyl; sowie auch für geradkettiges oder verzweigtes Alkyl mit 1 bis 4 Kohlenstoffatomen steht, wenn Y für Schwefel steht,

$X^1$ für Wasserstoff, Fluor, Chlor, Brom, Jod, Methyl, Ethyl, Isopropyl, tert.-Butyl, Methoxy, Methylthio, Methylsulfonyl, Trifluormethyl, Nitro, Cyano, Methoxycarbonyl, Ethoxycarbonyl, sowie für gegebenenfalls durch Fluor und Chlor substituiertes Phenyl steht;

$X^2$ für Wasserstoff, Fluor, Chlor, Methyl, Ethyl, Isopropyl, tert.-Butyl, Methoxy, Methylthio, sowie für Trifluormethyl steht;

$X^3$ für Wasserstoff, Fluor, Chlor sowie Methyl steht.

Verwendet man beispielsweise 1, 3-Bis-(4-chlorphenoxy)-1-brom-3-methyl-butan-2-on und 1, 2, 4-Triazol als Ausgangsstoffe, so kann der Reaktionsablauf durch das folgende Formelschema wiedergegeben werden:

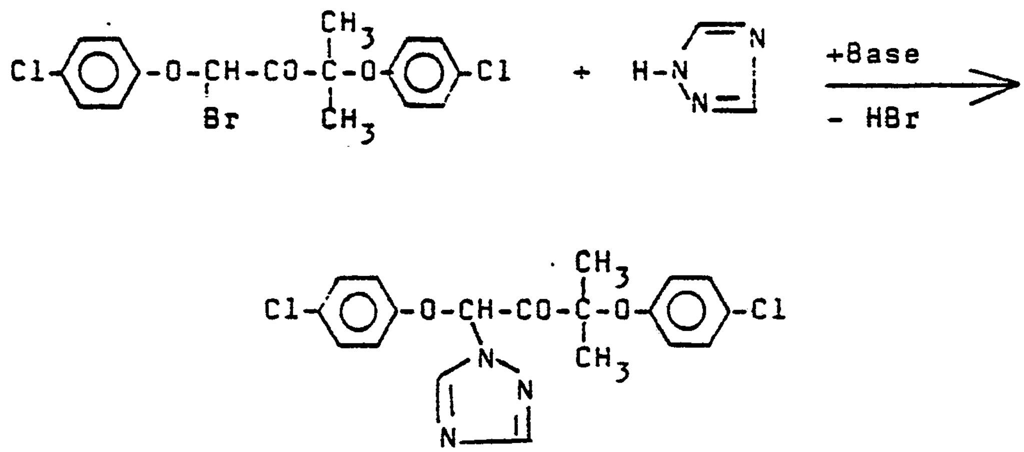


Verwendet man beispielsweise 1, 3-Bis-(4-chlorphenoxy)-3-methyl-1-(1, 2, 4-triazol-1-yl)-butan-2-on und Natriumborhydrid als Ausgangsstoffe, so kann der Reaktionsablauf durch das folgende Formelschema wiedergegeben werden:

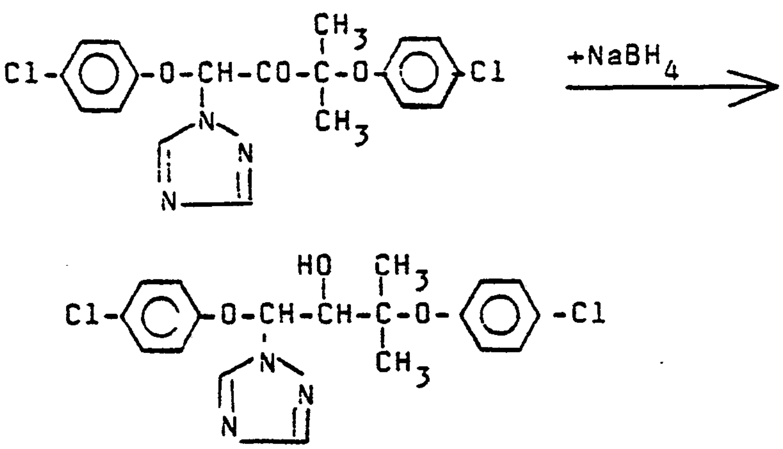


Die bei der Durchführung des erfindungsgemäßen Verfahrens als Ausgangsstoffe zu verwendenden Halogenetherketone sind durch die Formel (II) allgemein definiert. In dieser Formel stehen R, $X^1$, $X^2$, $X^3$ und Y vorzugsweise für diejenigen Reste, die bereits im Zusammenhang mit der Beschreibung der erfindungsgemäßen Stoffe der Formel (I) vorzugsweise für diese Substituenten genannt wurden.

Die Halogenetherketone der Formel (II) sind noch nicht bekannt. Sie können jedoch nach bekannten Verfahren erhalten werden, indem man z.B. bekannte Phenole der Formel

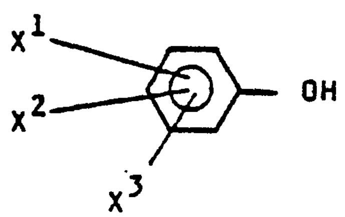

(III)

in welcher

$X^1$, $X^2$ und $X^3$ die oben angegebene Bedeutung haben,

mit einem Halogenketon der Formel

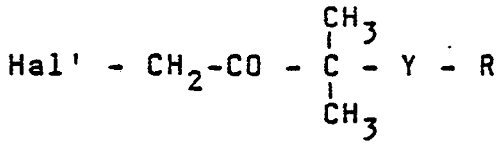

(IV)

in welcher
Y und R die oben angegebene Bedeutung haben und Hal' für Chlor oder Brom steht,
umsetzt. Das noch verbliebene aktive Wasserstoffatom wird anschließend in üblicher Weise gegen Halogen ausgetauscht (vergleiche auch die Herstellungsbeispiele). Die Halogenetherketone der Formel (II) können gegebenenfalls ohne Isolierung direkt weiter umgesetzt werden.

Die Halogenketone der Formel (IV) sind noch nicht bekannt. Sie sind jedoch Gegenstand einer eigenen älteren Anmeldung DE-A-30 48 266.7. Sie werden erhalten, indem man Ketone der Formel

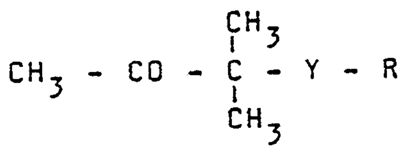

(V)

in welcher
R und Y die oben angegebene Bedeutung haben,
mit Chlor oder Brom in Gegenwart eines inerten organischen Lösungsmittels, wie beispielsweise Ether, chlorierten oder nichtchlorierten Kohlenwasserstoffen, bei Raumtemperatur umsetzt, oder mit üblichen Chlorierungsmitteln, wie beispielsweise Sulfurylchlorid, bei 20 bis 60°C umsetzt.

Die Ketone der Formel (V) sind teilweise bekannt (vergleiche z.B. US-Patentschrift 3 937 738), teilweise sind sie Gegenstand einer eigenen älteren Anmeldung DE-A-30 48 266. Sie können nach den dort angegebenen Verfahren erhalten werden, indem man z.B. Keto-Derivate der Formel

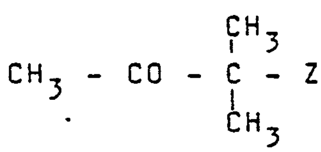

(VI)

in welcher
Z für Chlor Brom oder die Gruppierung $-O-SO_2-R^1$ steht, wobei
$R^1$ für Alkyl mit 1 bis 4 Kohlenstoffatomen sowie für gegebenenfalls subatituiertes Phenyl steht,
mit Verbindungen der Formel
Me - Y - R (VII)
in welcher
R und Y die oben angegebene Bedeutung haben und
Me für ein Alkalimetall, wie vorzugsweise Natrium und Kalium, oder Wasserstoff steht,
in Gegenwart eines organischen Lösungsmittels, wie beispielsweise Xylol, Glykol oder Dimethylformamid, und gegebenenfalls in Gegenwart eines Säurebindemittels, wie beispielsweise Natriumcarbonat, bei Temperaturen zwischen 80 und 150°C umsetzt; oder indem man z.B. Isopropyl-methylketon in üblicher Art und Weise mit Sulfenchloriden umsetzt.

Die Keto-Derivate der Formel (VI) sind bekannt bzw. können sie in allgemein bekannter Art und Weise erhalten werden.

Die Verbindungen der Formel (VII) sind allgemein bekannte Verbindungen der organischen Chemie und werden gegebenenfalls in situ eingesetzt.

Für die erfindungsgemäße Umsetzung kommen als Verdünnungsmittel inerte organische Lösungsmittel infrage. Hierzu gehören vorzugsweise Ketone, wie Diethylketon und insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; Benzol; Toluol; Formamide, wie insbesondere Dimethylformamid; und halogenierte Kohlenwasserstoffe.

Die erfindungsgemäße Umsetzung wird in Gegenwart eines Säurebindemittels vorgenommen. Man kann alle üblicherweise verwendbaren anorganischen oder organischen Säurebinder zugeben, wie Alkalicarbonate,

beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N, N-Dimethylcyclohexylamin, Dicyclohexylamin, N, N-Dimethylbenzylamin, weiterhin Pyridin und Diazabicyclooctan. Vorzugsweise verwendet man einen entsprechenden Überschuß an Azol.

Die Reaktionstemperaturen können in einem größeren Bereich variiert werden. Im allgemeinen arbeitet man zwischen etwa 20 bis etwa 150°C, vorzugsweise bei 60 bis 120°C. Bei Anwesenheit eines Lösungsmittels wird zweckmäßigerweise beim Siedepunkt des jeweiligen Lösungsmittels gearbeitet.

Bei der Durchführung des erfindungsgemäßen Verfahrens setzt man auf 1 Mol der Verbindung der Formel (II) vorzugsweise 2 bis 4 Mol Azol und 1 bis 4 Mol Säurebinder ein. Zur Isolierung der Verbindungen der Formel (I) wird das Lösungsmittel abdestilliert, der Rückstand mit einem organischen Solvens aufgenommen und mit Wasser gewaschen. Die organische Phase wird über Natriumsulfat getrocknet und im Vakuum vom Lösungsmittel befreit. Der Rückstand wird durch Destillation bzw. Umkristallisation oder Salzbildung und Umkristallisation gereinigt.

Die erfindungsgemäße Reduktion erfolgt in üblicher Weise, wie z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei 0 bis 30°C, vorzugsweise bei 0 bis 20°C durchgeführt. Hierzu setzt man auf 1 Mol des Ketons der Formel (Ia) etwa 1 Mol eines komplexen Hydrids, wie Natriumborhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösunsgmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden; im allgemeinen arbeitet man zwischen 20 und 120°C, vorzugsweise bei 50 bis 100°C.

Zur Durchführung der Reaktion setzt man auf 1 Mol des Ketons der Formel (Ia) etwa 0,3 bis 2 Mol Aluminiumisopropylat ein. Zur Isolierung der reduzierten Verbindungen der Formel (I) wird das überschüssige Lösungsmittel im Vakuum entfernt und die entstandenen Aluminiumverbindungen werden mit verdünnter Schwefelsäure oder Natronlauge zersetzt. Die weitere Aufarbeitung erfolgt in üblicher Weise.

Zur Herstellung von physiologisch verträglichen Säureadditionssalzen der Verbindungen der Formel (I) kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronenaäure, Salizylsäure, Sorbinsäure, Milchsäure, sowie Sulfonsäuren, wie z.B. p-Toluolsulfonsäure und 1, 5-Naphthalindisulfonsäure. Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösunsgmittel gereinigt werden.

Zur Herstellung von Metallsalz-Komplexen der Verbindungen der Formel (I) kommen vorzugsweise Salze von Metallen der II. bis IV. Haupt- und der I. bis II. sowie IV. bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) könnnen in einfacher Weise nach üblichen Verfahren erhalten werden, so z.B. durch Lösen des Metallsalzes in Alkohol, z.B. Ethanol und Hinzufügen zur Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen, so zur Bekämpfung von Erysiphe-Arten, wie z.B. gegen den Erreger des Gersten- bzw. Getreidemehltaus (Erysiphe graminis) und des Gurkenmehltaus (Erysiphe cichoracearum), oder zur Bekämpfung von Podosphaera-Arten, wie z.B. gegen

den Erreger des Apfelmehltaus (Podosphaera leucotricha); außerdem auch zur Bekämpfung von Reiskrankheiten, wie z.B. Pellicularia sasakii.

In entsprechenden Aufwandmengen zeigen die erfindungsgemäßen Stoffe auch pflanzenwachstumsregulierende Eigenschaften.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid. Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

**Herstellungsbeispiele**

**Beispiel 1**

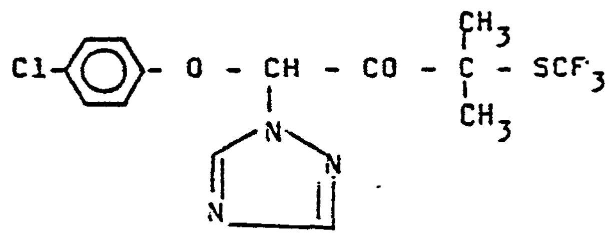

29 g (0,075 Mol) rohes 1-Brom-1-(4-chlorphenoxy)-3-methyl-3-trifluormethylthio-butan-2-on und 16 g (0,25 Mol) 1, 2, 4-Triazol werden in 150 ml Acetonitril 1 Stunde unter Rückfluß erhitzt. Danach wird eingeengt und der Rückstand mit Methylenchlorid/Wasser aufgenommen. Man trennt die organische Phase ab, trocknet über Natriumsulfat und engt erneut ein. Der ölige Rückstand wird chromatographiert. Man erhält 10,4 g (37 % der Theorie) 1-(4-Chlorphenoxy)-3-methyl-1-(1, 2, 4-triazol-1-yl)-3-trifluormethylthio-butan-2-on vom Schmelzpunkt 66° C.

Herstellung des Ausgangsproduktes

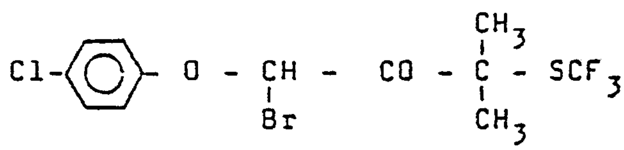

45 g (0,15 Mol) 1-(4-Chlorphenoxy)-3-methyl-3-trifluormethyl-thio-butan-2-on werden in 150 ml Chloroform gelöst und bei 20 °C tropfenweise so mit 7,5 ml (0,15 Mol) Brom versetzt, daß laufend Entfärbung eintritt. Nach beendeter Zugabe läßt man 20 Minuten bei 40 °C nachrühren und gibt das Reaktiongemisch anschließend in Wasser. Man extrahiert mit Chloroform und engt bei 40 °C ein. Man erhält quantitativ 1-Brom-1-(4-chlorphenoxy)-3-methyl-3-trifluormethylthio-butan-2-on, das direkt weiter umgesetzt wird.

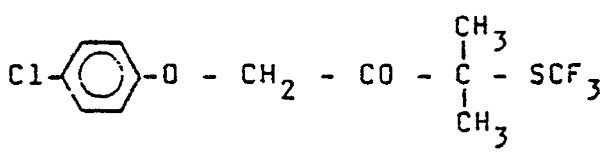

26 g (0,21 Mol) 4-Chlorphenol und 42 g (0,3 Mol) Kaliumcarbonat werden in 250 ml Aceton gegeben. Unter Rückfluß werden langsam 53 g (0,2 Mol) 1-Brom-3-methyl-3-trifluormethylthio-butan-2-on in 50 ml Aceton zugetropft. Nach beendeter Zugabe läßt man 12 Stunden unter Rückfluß rühren, filtriert und engt das Filtrat ein. Der Rückstand wird in 500 ml Methylenchlorid aufgenommen, mit Wasser und Natriumhydrogencarbonatlösung ausgeschüttelt, über Natriumsulfat getrocknet und eingeengt. Der Rückstand wird im Vakuum destilliert. Man erhält 45,3 g (73 % der Theorie) 1-(4-Chlorpnenoxy)-3-methyl-3-trifluormethylthio-butan-2-on vom Siedepunkt 105° C/0,4 mbar.

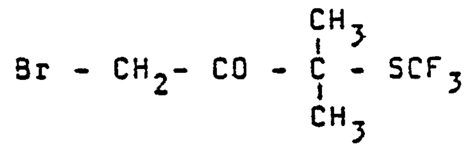

Durch Umsetzung von 3-Methyl-3-trifluormethylthio-butan-2-on mit Brom in der oben beschriebenen Art und Weise erhält man in 85%-iger Ausbeute 1-Brom-3-methyl-3-trifluormethylthio-butan-2-on vom Siedepunkt 56° C/0,4 mbar.

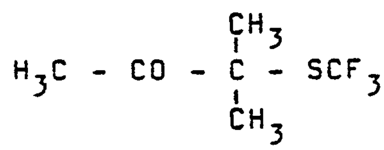

In einem schmalen Rührgefäß mit Kühler werden 334 g Methylisopropylketon in 1,2 l Methylenchlorid vorgelegt.

Durch ein bis zum Boden des Gefäßes reichendes Einleitungsrohr werden bei Raumtemperatur innerhalb von 4 Stunden 220 g Trifluormethan-sulfenchlorid eingeleitet. Man läßt das Reaktionsgemisch über Nacht stehen. Anschließend wird destilliert. Man erhält 242g (80,7% der Theorie) rohes 3-Methyl-3-trifluormethylthio-butan-2-on, das durch fraktionierte Destillation gereinigt wird:

Siedepunkt: 60° C/50 mbar und Brechungsindex

$n_D^{20} = 1,4030$

**Beispiel 2**

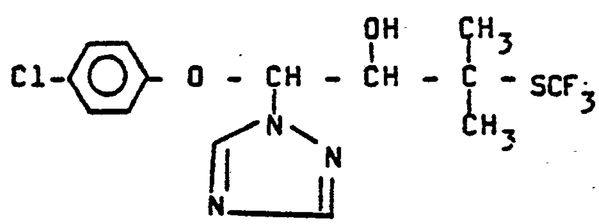

10 g (0,026Mol) 1-(4-Chlorphenoxy)-3-methyl-1-(1, 2, 4-triazol-1-yl)-3-trifluormethylthio-butan-2-on (Beispiel 1) werden in 100 ml Methanol gelöst und portionsweise mit 1 g (0,025 Mol) Natriumborhydrid versetzt. Man läßt 30 Minuten bei Raumtemperatur nachrühren und gibt das Reaktionsgemisch danach in Wasser. Man extrahiert mit Methylenchlorid, trocknet über Natriumsulfat und engt im Vakuum ein. Der Rückstand kristallisiert nach kurzer Zeit. Man erhält 9,5 g (95% der Theorie) 1-(4-Chlorphenoxy)-3-methyl-1-(1, 2, 4-triazol-1-yl)-3-trifluormethylthio-butan-2-ol vom Schmelzpunkt 85-87° C.

In entsprechender Weise werden die nachfolgenden Beispiele der allgemeinen Formel

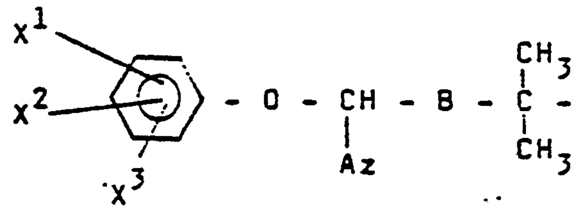 (I)

erhalten:

| Bsp. Nr. | $X^1$ | $X^2$ | $X^3$ | Az | B | -YR | Schmelzpunkt (°C) |
|---|---|---|---|---|---|---|---|
| 3 | 4-Cl | H | H | 1,2,4-Triazol-1-yl | CO | 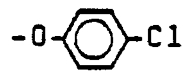 | Harz |
| 4 | 4-Cl | H | H | 1,2,4-Triazol-1-yl | CO | 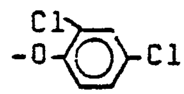 | Harz |
| 5 | 4-Cl | H | H | 1,2,4-Triazol-1-yl | CO | 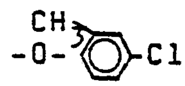 | Harz |
| 6 | 4-Cl | H | H | 1,2,4-Triazol-1-yl | CO | 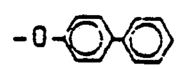 | 110 |
| 7 | 2-Cl | 4-Cl | H | 1,2,4-Triazol-1-yl | CO | $-SCF_3$ | 105 |
| 8 | $2-CH_3$ | 4-Cl | H | 1,2,4-Triazol-1-yl | CO | $-SCF_3$ | 90 |
| 9 | 4-Cl | H | H | 1,2,4-Triazol-4-yl | CO | 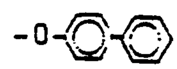 | Harz |
| 10 | 2-Cl | 4-Cl | H | 1,2,4-Triazol-4-yl | CO | $-SCF_3$ | 156 |
| 11 | $2-CH_3$ | 4-Cl | H | 1,2,4-Triazol-4-yl | CO | $-SCF_3$ | 141 |
| 12 | 2-Cl | 4-Cl | H | 1,2,4-Triazol-4-yl | CO | 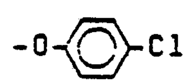 | 161 |
| 13 | 4-Cl | H | H | 1,2,4-Triazol-1-yl | CH(OH) | 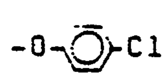 | Harz |
| 14 | 2-Cl | 4-Cl | H | 1,2,4-Triazol-1-yl | CH(OH) | $-SCF_3$ | 91 |

| Bsp. Nr. | $X^1$ | $X^2$ | $X^3$ | Az | B | -YR | Schmelzpunkt(°C) |
|---|---|---|---|---|---|---|---|
| 15 | 2-Cl | 4-Cl | H | 1,2,4-Triazol-1-yl | CH(OH) | 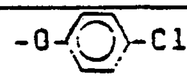 | Harz |
| 16 | 4-Cl | H | H | Imidazol-1-yl | CO | 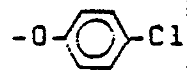 | Harz |
| 17 | 4-Cl | H | H | Imidazol-1-yl | CO | $-SCF_3$ | 88 |
| 18 | 4-Cl | H | H | Imidazol-1-yl | CO | 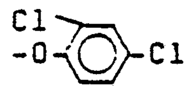 | Harz |
| 19 | 4-Cl | H | H | Imidazol-1-yl | CO | 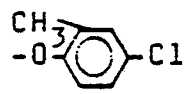 | Harz |
| 20 | 4-Cl | H | H | Imidazol-1-yl | CO | 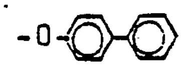 | Harz |
| 21 | 2-Cl | 4-Cl | H | Imidazol-1-yl | CO | $-SCF_3$ | 105-07 |
| 22 | $2-CH_3$ | 4-Cl | H | Imidazol-1-yl | CO | $-SCF_3$ | Oel |
| 23 | 2-Cl | 4-Cl | H | Imidazol-1-yl | CO | 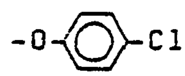 | Harz |
| 24 | 4-Cl | H | H | Imidazol-1-yl | CO | 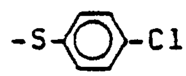 | 150 |
| 25 | 4-Cl | H | H | Imidazol-1-yl | CH(OH) | $-SCF_3$ | 145 |
| 26 | 4-Cl | H | H | Imidazol-1-yl | CH(OH) | 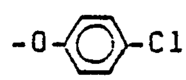 | Harz |
| 27 | 4-Cl | H | H | Imidazol-1-yl | CH(OH) | 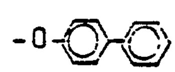 | 163 |
| 28 | 4-Cl | H | H | Imidazol-1-yl | CH(OH) | 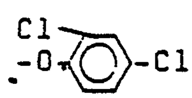 | Harz |
| 29 | 2-Cl | 4-Cl | H | Imidazol-1-yl | CH(OH) | $-SCF_3$ | 137-39 |

| Bsp. Nr. | $X^1$ | $X^2$ | $X^3$ | Az | B | -YR | Schmelzpunkt ($^oC$) |
|---|---|---|---|---|---|---|---|
| 30 | 4-Cl | H | H | Imidazol-1-yl | CH(OH) | 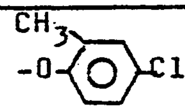 | Harz |
| 31 | 4-Cl | H | H | Imidazol-1-yl | CH(OH) | 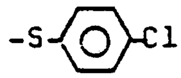 | 145-147 |
| 32 | 4-Cl | H | H | 1,2,4-Triazol-1-yl | CO | 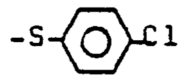 | 116 |
| 33 | 4-Cl | H | H | 1,2,4-Triazol-4-yl | CO | 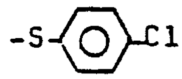 | 156 |
| 34 | 2-Cl | 4-Cl | H | Imidazol-1-yl | CO | 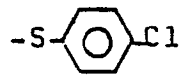 | 133 |
| 35 | 2-Cl | 4-Cl | H | 1,2,4-Triazol-1-yl | CO | 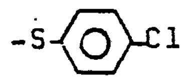 | 90- 94 |
| 36 | 2-Cl | 4-Cl | H | 1,2,4-Triazol-4-yl | CO | 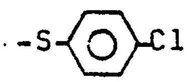 | Harz |
| 37 | 4-F | H | H | Imidazol-1-yl | CO | $-SCF_3$ | 68- 70 |
| 38 | 4-Cl | H | H | 1,2,4-Triazol-1-yl | CH(OH) | 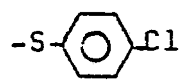 | 45 |
| 39 | $2-CH_3$ | 4-Cl | H | Imidazol-1-yl | CH(OH) | $-SCF_3$ | 115 |
| 40 | 4-F | H | H | Imidazol-1-yl | CH(OH) | $-SCF_3$ | 120 |
| 41 | 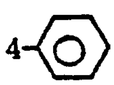 | H | H | 1,2-4-Triazol-1-yl | CH(OH) | $-SCF_3$ | 98-100 |
| 42 | 2-Cl | 4-Cl | H | Imidazol-1-yl | CH(OH) | 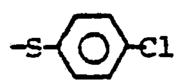 | Harz |
| 43 | 4-F | H | H | Imidazol-1-yl | CO | 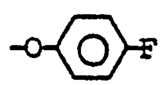 | Harz |

| | $x^1$ | $x^2$ | $x^3$ | Az | B | -YR | |
|---|---|---|---|---|---|---|---|
| 44 | 4-F | H | H | 1,2,4-Tri-azol-1-yl | CO | 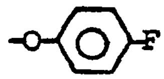 | 78-80 |
| 45 | 4-F | H | H | Imidazol-1-yl | CH(OH) | 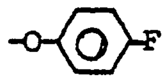 | Harz |
| 46 | 4-F | H | H | 1,2,4-Tri-azol-1-yl | CH(OH) | 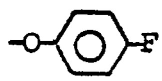 | Harz |
| 47 | 4-Cl | H | H | Imidazol-1-yl | CO | 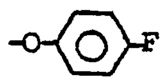 | Harz |
| 48 | 4-Cl | H | H | 1,2,4-Tri-azol-1-yl | CO | 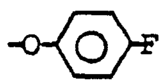 | 89 |
| 49 | 4-Cl | H | H | 1,2,4-Tri-azol-4-yl | CO | 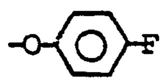 | 80-85 |
| 50 | 4-Cl | H | H | 1,2,4-Tri-azol-1-yl | CH(OH) | 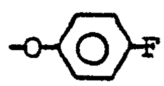 | Harz |

**Verwendungsbeispiele**

In den nachfolgenden Beispielen werden die nachstehend angegebenen Verbindungen als Vergleichssubstanzen eingesetzt:

(A) 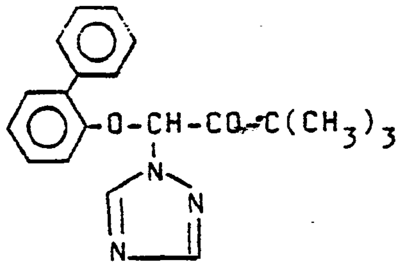

(B) 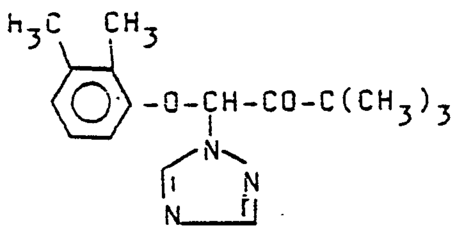

(C) 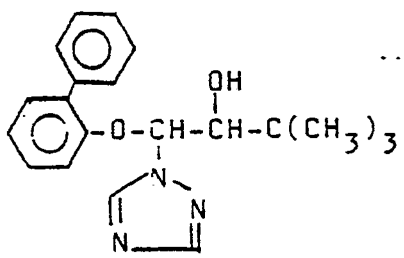

(D) 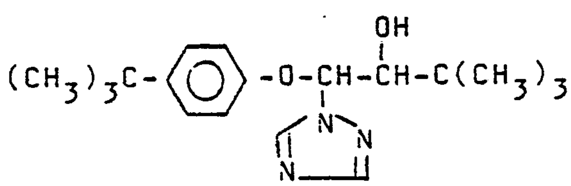

(E) 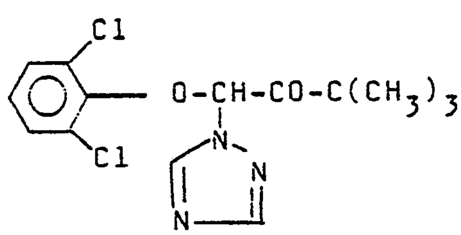

(F) 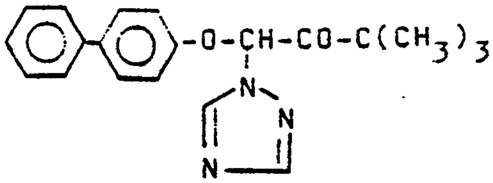

(G) 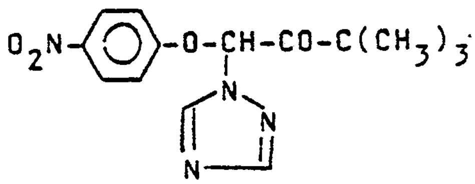

**Beispiel A**

Erysiphe-Test (Gerste) / protektiv
Lösungsmittel: 100 Gewichtsteile Dimethylformamid
Emulgator: 0,25 Gewichtsteile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20° C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele:
1, 4, 6, 7, 8, 12, 2, 17, 18, 21, 22 und 29.

**Beispiel B**

Erysiphe-Test (Gerste) / Saatgutbehandlung
Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Die Gerste sät man mit 3 x 12 Korn 2 cm tief in eine Standarderde. 7 Tage nach der Aussaat, wenn die jungen Pflanzen ihr erstes Blatt entfaltet haben, werden sie mit Sporen von Erysiphe graminis f. sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 1, 4, 10 und 2.

**Beispiel C**

Sphaerotheca-Test (Gurke) / protektiv
Lösungsmittel: 4,7 Gewichtsteile Aceton
Emulgator: 0,3 Gewichtsteile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca.75 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 17 und 21.

**Patentansprüche**

1. 3-Substituierte 1-Azolyl-3-methyl-1-phenoxy-butan-2-one und -ole der allgemeinen Formel

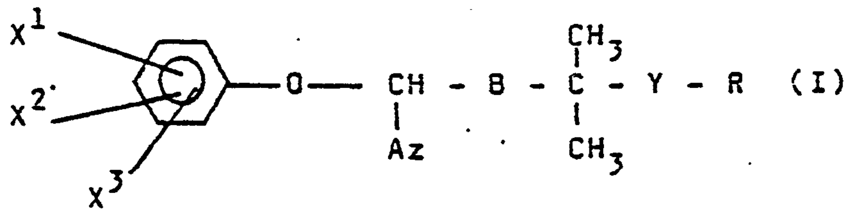

in welcher
Az für 1, 2, 4-Triazol-1-yl, 1, 2, 4-Triazol-4-yl oder Imidazol-1-yl steht,
B für die Ketogruppe oder die CH(OH)-Gruppe steht,
Y für Sauerstoff oder Schwefel steht,
R für Halogenalkyl mit 1 bis 4 Kohlenstoffatomen und 1 bis 5 gleichen oder verschiedenen Halogenatomen, sowie für gegebenenfalls substituiertes Phenyl und Benzyl steht, wobei als Phenylsubstituenten jeweils Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoffatomen und 1 bis 5 gleichen oder verschiedenen Halogenatomen, Nitro, Cyano, Alkoxycarbonyl mit 1 bis 4 Kohlenstoffatomen im Alkylteil, sowie gegebenenfalls durch Halogen substituiertes Phenyl zu nennen sind; sowie auch für geradkettiges oder verzweigtes Alkyl mit 1 bis 6 Kohlenstoffatomen steht, wenn Y für Schwefel steht;
$X^1$ für Wasserstoff, Halogen, für Alkyl, Alkoxy, Alkylthio und Alkylsulfonyl mit jeweils 1 bis 4 Kohlenstöffatomen, für Halogenalkyl mit 1 bis 4 Kohlenstoffatomen und 1 bis 5 gleichen oder verschiedenen Halogenatomen, für Nitro, Cyano, Alkoxycarbonyl mit 1 bis 4 Kohlenstoffatomen im Alkylteil, sowie für gegebenenfalls durch Halogen substituiertes Phenyl steht;
$X^2$ für Wasserstoff, Halogen, für Alkyl, Alkoxy, Alkylthio und Alkylsulfonyl mit jeweils 1 bis 4 Kohlenstoffatomen, für Halogenalkyl mit 1 bis 4 Kohlenstoffatomen und 1 bis 5 gleichen oder verschiedenen Halogenatomen, für Nitro, Cyano, Alkoxycarbonyl mit 1 bis 4 Kohlenstoffatomen im Alkylteil, sowie für gegebenenfalls durch Halogen substituiertes Phenyl steht,
$X^3$ für Wasserstoff, Halogen, für Alkyl, Alkoxy und Alkylthio mit jeweils 1 bis 4 Kohlenstoffatomen, sowie für Halogenalkyl mit 1 bis 4 Kohlenstoffatomen und 1 bis 5 gleichen oder verschiedenen Halogenatomen steht;
sowie deren physiologisch verträglichen Säureadditions-Salzen und Metallsalz-Komplexe.

2) Verbindungen der allgemeinen Formel (I) in Anspruch 1,
in welcher
R für Halogenalkyl mit 1 bis 2 Kohlenstoffatomen und 1 bis 5 gleichen oder verschiedenen Fluor- oder Chloratomen steht, sowie für gegebenenfalls substituiertes Phenyl oder Benzyl steht, wobei als Phenylsubstituenten jeweils genannt seien: Fluor, Chlor, Methyl, Ethyl, Isopropyl, tert.-Butyl, Methoxy, Methylthio, Trifluormethyl, Trifluormethoxy, Trifluormethylthio, Nitro, Cyano, sowie gegebenenfalls durch Fluor und Chlor substituiertes Phenyl; sowie auch für geradkettiges oder verzweigtes Alkyl mit 1 bis 4 Kohlenstoffatomen steht, wenn Y für Schwefel steht,
$X^1$ für Wasserstoff, Fluor, Chlor, Brom, Jod, Methyl, Ethyl, Isopropyl, tert.-Butyl, Methoxy, Methylthio, Methylsulfonyl, Trifluormethyl, Nitro, Cyano, Methoxycarbonyl, Ethoxycarbonyl, sowie für gegebenenfalls durch Fluor und Chlor substituiertes Phenyl steht;
$X^2$ für Wasserstoff, Fluor, Chlor, Methyl, Ethyl, Isopropyl, tert.-Butyl, Methoxy, Methylthio, sowie für Trifluormethyl steht;
$X^3$ für Wasserstoff, Fluor, Chlor sowie Methyl steht.

3) Verfahren zur Herstellung von 3-substituierten 1-Azolyl-3-methyl-1-phenoxy-butan-2-onen und -olen der allgemeinen Formel

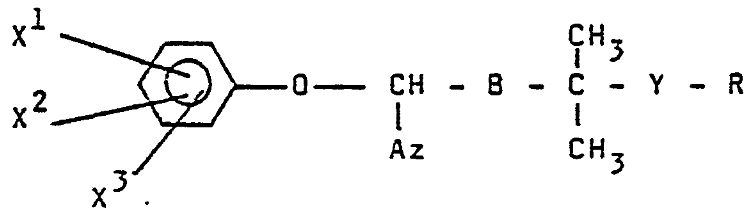 (I)

in welcher
Az für 1, 2, 4-Triazol-1-yl, 1, 2, 4-Triazol-4-yl oder Imidazol-1-yl steht,
B für die Ketogruppe oder die CH(OH)-Gruppe steht,
Y für Sauerstoff oder Schwefel steht,
R für Halogenalkyl mit 1 bis 4 Kohlenstoffatomen und 1 bis 5 gleichen oder verschiedenen Halogenatomen, sowie für gegebenenfalls substituiertes Phenyl und Benzyl steht, wobei als Phenylsubstituenten jeweils Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoffatomen und 1 bis 5 gleichen oder verschiedenen Halogenatomen, Nitro, Cyano, Alkoxycarbonyl mit 1 bis 4 Kohlenstoffatomen im Alkylteil, sowie gegebenenfalls durch Halogen substituiertes Phenyl zu nennen sind; sowie auch für geradkettiges oder verzweigtes Alkyl mit 1 bis 6 Kohlenstoffatomen steht, wenn Y für Schwefel steht;
$X^1$ für Wasserstoff, Halogen, für Alkyl, Alkoxy, Alkylthio und Alkylsulfonyl mit jeweils 1 bis 4 Kohlenstoffatomen, für Halogenalkyl mit 1 bis 4 Kohlenstoffatomen und 1 bis 5 gleichen oder verschiedenen Halogenatomen, für Nitro, Cyano, Alkoxycarbonyl mit 1 bis 4 Kohlenstoffatomen im Alkylteil, sowie für gegebenenfalls durch Halogen substituiertes Phenyl steht;
$X^2$ für Wasserstoff, Halogen, für Alkyl, Alkoxy, Alkylthio und Alkylsulfonyl mit jeweils 1 bis 4 Kohlenstoffatomen, für Halogenalkyl mit 1 bis 4 Kohlenstoffatomen und 1 bis 5 gleichen oder verschiedenen Halogenatomen, für Nitro, Cyano, Alkoxycarbonyl mit 1 bis 4 Kohlenstoffatomen im Alkylteil, sowie für gegebenenfalls durch Halogen substituiertes Phenyl steht,
$X^3$ für Wasserstoff, Halogen, für Alkyl, Alkoxy und Alkylthio mit jeweils 1 bis 4 Kohlenstoffatomen, sowie für Halogenalkyl mit 1 bis 4 Kohlenstoffatomen und 1 bis 5 gleichen oder verschiedenen Halogenatomen steht;
sowie deren physiologisch verträglichen Säureadditions-Salzen und Metallsalz-Komplexen, dadurch gekennzeichnet, daß man Halogenetherketone der Formel

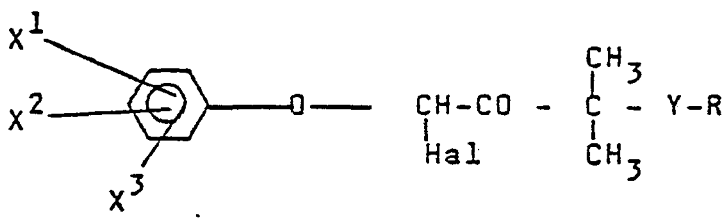 (II)

in welcher Hal für Halogen steht und
R, $X^1$, $X^2$, $X^3$ und Y die oben angegebene Bedeutung haben,
mit 1, 2, 4-Triazol oder Imidazol in Gegenwart eines Verdünnungsmittels und in Gegenwart eines Säurebindemittels umsetzt, und gegebenenfalls noch die so erhaltenen Keto-Derivate der Formel

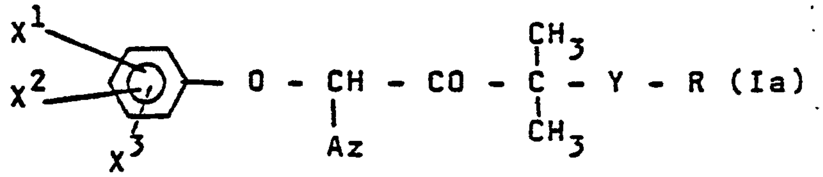

in welcher
Az, R, $X^1$, $X^2$, $X^3$ und Y die oben angegebene Bedeutung haben,
nach bekannten Methoden in üblicher Weise reduziert, und gegebenenfalls an die so erhaltenen Verbindungen der Formel (I) anschließend eine Säure oder ein Metallsalz addiert.

4) Fungizide Mittel, gekennzeichnet durch einen Gehalt an mindestens einem 3-substituierten 1-Azolyl-3-methyl-1-phenoxy-butan-2-on oder -ol der Formel (I) in Ansprüchen 1 und 3.

5. Verwendung von 3-substituierten 1-Azolyl-3-methyl-1-phenoxy-butan-2-onen oder -olen der Formel (I) in Ansprüchen 1 und 3 zur Bekämpfung von Pilzen.

**Claims**

1. 3-Substituted 1-azolyl-3-methyl-1-phenoxy-butan-2-ones and -ols of the general formula

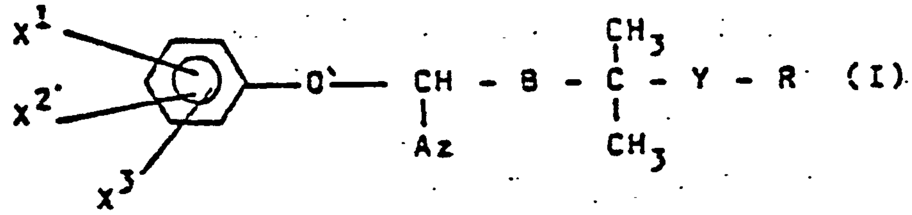

in which
Az represents 1, 2, 4-triazol-1-yl, 1, 2, 4-triazol-4-yl or imidazol-1-yl,
B represents the keto group or the CH(OH) group,
Y represents oxygen or sulphur,
R represents halogenoalkyl with 1 to 4 carbon atoms and 1 to 5 identical or different halogen atoms, and optionally substituted phenyl and benzyl, the phenyl substituents to be mentioned in each case being halogen, alkyl, alkoxy and alkylthio with in each case 1 to 4 carbon atoms, halogenoalkyl, halogenoalkoxy and halogenoalkylthio with in each case 1 to 4 carbon atoms and 1 to 5 identical or different halogen atoms, nitro, cyano, alkoxycarbonyl with 1 to 4 carbon atoms in the alkyl part, and optionally halogen-substituted phenyl; and also represents straight-chain or branched alkyl with 1 to 6 carbon atoms when Y represents sulphur;
$X^1$ represents hydrogen, halogen, alkyl, alkoxy, alkylthio and alkylsulphonyl with in each case 1 to 4 carbon atoms, halogenoalkyl with 1 to 4 carbon atoms and 1 to 5 identical or different halogen atoms, nitro, cyano, alkoxycarbonyl with 1 to 4 carbonatoms in the alkyl part, and optionally halogensubstituted phenyl;
$X^2$ represents hydrogen, halogen, alkyl, alkoxy, alkylthio and alkylsulphonyl with in each case 1 to 4 carbon atoms, halogenoalkyl with 1 to 4 carbon atoms and 1 to 5 identical or different halogen atoms, nitro, cyano, alkoxycarbonyl with 1 to 4 carbon atoms in the alkyl part, and optionally halogen-substituted phenyl, and
$X^3$ represents hydrogen, halogen, alkyl, alkoxy and alkylthio with in each case 1 to 4 carbon atoms and halogenoalkyl with 1 to 4 carbon atoms and 1 to 5 identical or different halogen atoms;
and physiologically tolerated acid addition salts and metal salt complexes thereof.

2. Compounds of the general formula (I) in Claim 1, in which R represents halogenoalkyl with 1 to 2 carbon atoms and 1 to 5 identical or different fluorine or chlorine atoms, and optionally substituted phenyl or benzyl, the following being mentioned as the phenyl substituents in each case: fluorine, chlorine, methyl, ethyl, isopropyl, tert.-butyl, methoxy, methylthio, trifluoromethyl, trifluoromethoxy, trifluoromethylthio, nitro, cyano, and optionally fluorine and chlorine-substituted phenyl; and also represents straight-chain or branched alkyl with 1 to 4 carbon atoms, when Y represents sulphur,

$X^1$ represents hydrogen, fluorine, chlorine, bromine, iodine, methyl, ethyl, isopropyl, tert.-butyl, methoxy, methylthio, methylsulphonyl, trifluoromethyl, nitro, cyano, methoxycarbonyl, ethoxycarbonyl and optionally fluorine- and chlorine-substituted phenyl;

$X^2$ represents hydrogen, fluorine, chlorine, methyl, ethyl, isopropyl, tert.-butyl, methoxy, methylthio, and trifluoromethyl; and

$X^3$ represents hydrogen, fluorine, chlorine and methyl.

3. Process for the preparation of 3-substituted 1-azolyl-3-methyl-1-phenoxy-butan-2-ones and -ols of the general formula

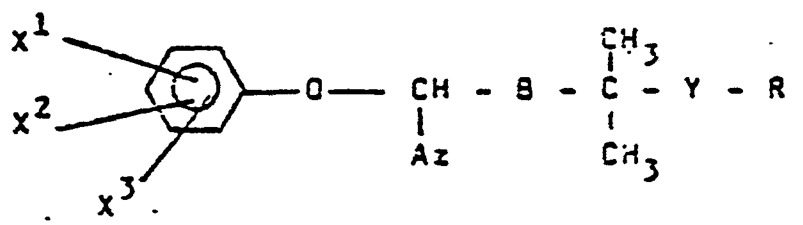
(I)

in which

Az represents 1, 2, 4-triazol-1-yl, 1, 2, 4-triazol-4-yl or imidazol-1-yl,

B represents the keto group or the CH(OH) group,

Y represents oxygen or sulphur,

R represents halogenoalkyl with 1 to 4 carbon atoms and 1 to 5 identical or different halogen atoms, and optionally substituted phenyl and benzyl, the phenyl substituents to be mentioned in each case being halogen, alkyl, alkoxy and alkylthio with in each case 1 to 4 carbon atoms, halogenoalkyl, halogenoalkoxy and halogenoalkylthio with in each case 1 to 4 carbon atoms and 1 to 5 identical or different halogen atoms, nitro, cyano, alkoxycarbonyl with 1 to 4 carbon atoms in the alkyl part, and optionally halogen-substituted phenyl; and also represents straight-chain or branched alkyl with 1 to 6 carbon atoms when Y represents sulphur;

$X^1$ represents hydrogen, halogen, alkyl, alkoxy, alkylthio and alkylsulphonyl with in each case 1 to 4 carbon atoms, halogenoalkyl with 1 to 4 carbon atoms and 1 to 5 identical or different halogen atoms, nitro, cyano, alkoxycarbonyl with 1 to 4 carbonatoms in the alkyl part, and optionally halogensubstituted phenyl;

$X^2$ represents hydrogen, halogen, alkyl, alkoxy, alkylthio and alkylsulphonyl with in each case 1 to 4 carbon atoms, halogenoalkyl with 1 to 4 carbon atoms and 1 to 5 identical or different halogen atoms, nitro, cyano, alkoxycarbonyl with 1 to 4 carbon atoms in the alkyl pärt, and optionally halogen-substituted phenyl, and

$X^3$ represents hydrogen, halogen, alkyl, alkoxy and alkylthio with in each case 1 to 4 carbon atoms and halogenoalkyl with 1 to 4 carbon atoms and 1 to 5 identical or different halogen atoms;

and physiologically tolerated acid addition salts and metal salt complexes thereof, characterised in that halogenoether ketones of the formula

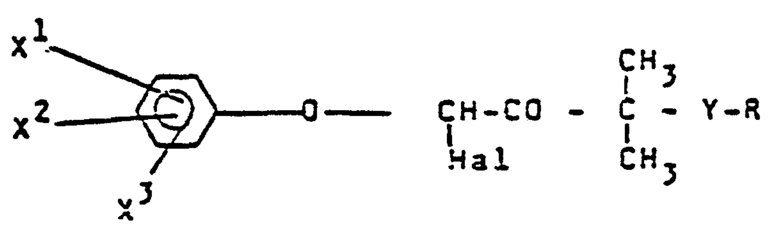
(II)

in which Hal represents halogen and

R, $X^1$, $X^2$, $X^3$, and Y have the meaning given above,

are reacted with 1, 2, 4-triazole or imidazole in the presence of a diluent and in the presence of an acid-binding agent, and, if desired, the keto derivatives thus obtained, of the formula

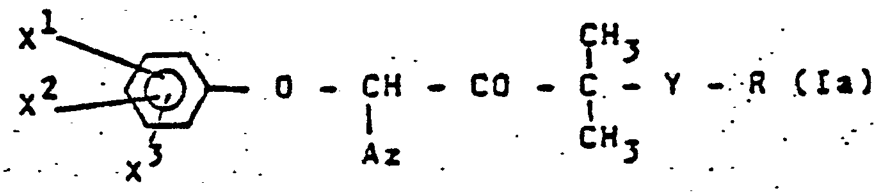

in which
Az, R, $X^1$, $X^2$, $X^3$, and Y have the meaning given above,
are then reduced in a customary manner by known methods, and, if desired, an acid or a metal salt is then added on to the compounds thus obtained, of the formula (I).

4. Fungicidal agents, characterised in that they contain at least one 3-substituted 1-azolyl-3-methyl-1-phenoxy-butan-2-one or -ol of the formula (I) in Claims 1 and 3.

5. Use of 3-substituted 1-azolyl-3-methyl-1-phenoxy-butan-2-ones or -ols of the formula (I) in Claims 1 and 3 for combating fungi.

**Revendications**

1. 1-azolyl-3-méthyl-1-phénoxy-butane-2-ones et -ols substitués en position 3, de formule générale

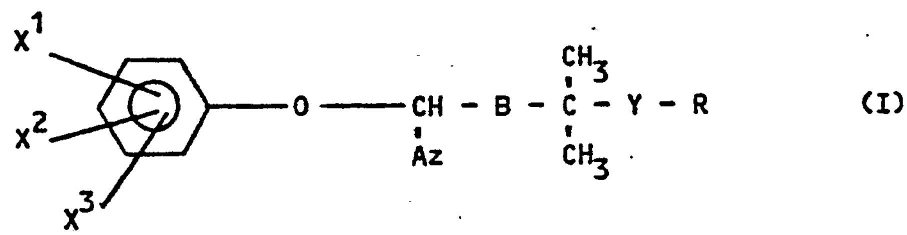

dans laquelle
Az représente un groupe 1, 2, 4-triazole-1-yle, 1, 2, 4-triazole-4-yle ou imidazole-1-yle,
B représente le groupe céto ou le groupe CH(OH),
Y représente l'oxygène ou le soufre,
R représente un groupe halogénoalkyle contenant 1 à 4 atomes de carbone et 1 à 5 atomes d'halogènes identiques ou différents, ou un groupe phényle ou benzyle éventuellement substitué, les substituants du groupe phényle étant des halogènes, des groupes alkyles, alcoxy et alkylthio contenant chacun 1 à 4 atomes de carbone, halogénoalkyle, halogénoalcoxy ou halogénoalkylthio contenant chacun 1 à 4 atomes de carbone et 1 à 5 atomes d'halogènes identiques ou différents, nitro, cyano, alcoxycarbonyle contenant 1 à 4 atomes de carbone dans la partie alkyle, ou encore phényle éventuellement substitué par des halogènes; ou bien un groupe alkyle à chaîne droite ou ramifiée en $C_1$-$C_6$ lorsque Y représente le soufre;
$X^1$ représente l'hydrogène, un halogène, un groupe alkyle, alcoxy, alkylthio ou alkylsulfonyle contenant chacun 1 à 4 atomes de carbone, halogénoalkyle contenant 1 à 4 atomes de carbone et 1 à 5 atomes d'halogènes identiques ou différents, nitro, cyano, alcoxycarbonyle contenant 1 à 4 atomes de carbone dans la partie alkyle, ou phényle éventuellement substitué par des halogènes;
$X^2$ représente l'hydrogène, un halogène, un groupe alkyle, alcoxy, alkylthio ou alkylsulfonyle contenant chacun 1 à 4 atomes de carbone, halogénoalkyle contenant 1 à 4 atomes de carbone et 1 à 5 atomes d'halogènes identiques ou différents, nitro, cyano, alcoxycarbonyle contenant 1 à 4 atomes de carbone dans la partie alkyle, ou phényle éventuellement substitué par des halogènes,
$X^3$ représente l'hydrogène, un halogène, un groupe alkyle, alcoxy ou alkylthio contenant chacun 1 à 4 atomes de carbone, ou halogénoalkyle contenant 1 à 4 atomes de carbone et 1 à 5 atomes d'halogènes identiques ou

différents;

Leurs sels formés par addition avec des acides et complexes de sels métalliques tolérés par l'organisme.

2. Composés de formule générale (I) de la revendication 1, dans lesquels:

R représente un groupe halogénoalkyle contenant 1 à 2 atomes de carbone et 1 à 5 atomes identiques ou différents de fluor ou de chlore, ou un groupe phényle ou benzyle éventuellement substitué, les substituants du groupe phényle étant le fluor, le chlore, les groupes méthyle, éthyle, isopropyle, tert-butyle, méthoxy, méthylthio, trifluorométhyle, trifluorométhoxy, trifluorométhylthio, nitro, cyano, ou phényle éventuellement substitué par le fluor et le chlore; ou encore un groupe alkyle à chaîne droite ou ramifiée en $C_1$-$C_4$ lorsque Y représente le soufre,

$X^1$ représente l'hydrogène, le fluor, le chlore, le brome, l'iode, un groupe méthyle, éthyle, isopropyle, tert-butyle, méthoxy, méthylthio, méthylsulfonyle, trifluorométhyle, nitro, cyano, méthoxycarbonyle, éthoxycarbonyle, ou phényle éventuellement substitué par le fluor et le chlore;

$X^2$ représente l'hydrogène, le fluor, le chlore, un groupe méthyle, éthyle, isopropyle, tert-butyle, méthoxy, méthylthio ou trifluorométhyle;

$X^3$ représente l'hydrogène, le fluor, le chlore ou un groupe méthyle.

3. Procédé de préparation des 1-azolyl-3-méthyl-1-phénoxy-butane-2-ones et -ols substitués en position 3 de formule générale:

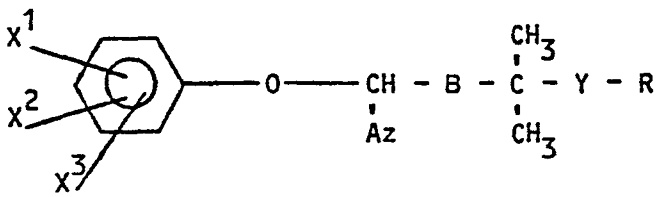

(I)

dans laquelle

Az représente un groupe 1, 2, 4-triazole-1-yle, 1, 2, 4-triazole-4-yle ou imidazole-1-yle,

B représente le groupe céto ou le groupe CH(OH),

Y représente l'oxygène ou le soufre,

R représente un groupe halogénoalkyle contenant 1 à 4 atomes de carbone et 1 à 5 atomes d'halogènes identiques ou différents, ou un groupe phényle ou benzyle éventuellement substitué, les substituants du groupe phényle étant des halogènes, des groupes alkyle, alcoxy et alkylthio contenant chacun 1 à 4 atomes de carbone, halogénoalkyle, halogénoalcoxy ou halogénoalkylthio contenant chacun 1 à 4 atomes de carbone et 1 à 5 atomes d'halogènes identiques ou différents, nitro, cyano, alcoxycarbonyle contenant 1 à 4 atomes de carbone dans la partie alkyle, ou encore phényle éventuellement substitué par des halogènes, ou bien un groupe alkyle à chaîne droite ou ramifiée en $C_1$-$C_6$ lorsque Y représente le soufre;

$X^1$ représente l'hydrogène, un halogène, un groupe alkyle, alcoxy, alkylthio ou alkylsulfonyle contenant chacun 1 à 4 atomes de carbone, halogénoalkyle contenant 1 à 4 atomes de carbone et 1 à 5 atomes d'halogènes identiques ou différents, nitro, cyano, alcoxycarbonyle contenant 1 à 4 atomes de carbone dans la partie alkyle, ou phényle éventuellement substitué par des halogènes

$X^2$ représente l'hydrogène, un halogène, un groupe alkyle, alcoxy, alkylthio ou alkylsulfonyle contenant chacun 1 à 4 atomes de carbone, halogénoalkyle contenant 1 à 4 atomes de carbone et 1 à 5 atomes de d'halogènes identiques ou différents, nitro, cyano, alcoxycarbonyle contenant 1 à 4 atomes de carbone dans la partie alkyle, ou phényle éventuellement substitué par des halogènes,

$X^3$ représente l'hydrogène, un halogène, un groupe alkyle, alcoxy ou alkylthio contenant chacun 1 à 4 atomes de carbone, ou halogénoalkyle contenant 1 à 4 atomes de carbone et 1 à 5 atomes d'halogènes identiques ou différents;

et de leurs sels formes par addition avec des acides et complexes de sels métalliques tolérés par l'organisme, caractérisé en ce que l'on fait réagir des halogéno-éther-cétones de formule:

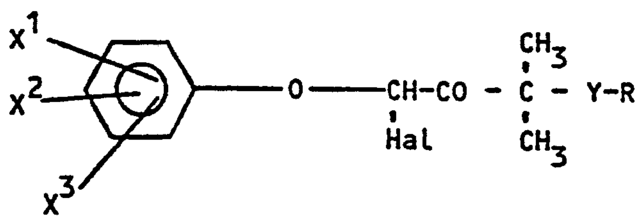

(II)

dans laquelle ⊁ représente un halogène et
R, $X^1$, $X^2$, $X^3$ et Y ont les significations indiquées ci-dessus,
avec le 1, 2, 4-triazole ou l'imidazole en présence d'un diluant et en présence d'un agent fixant les acides, ce qui donne des dérivés cétoniques de formule

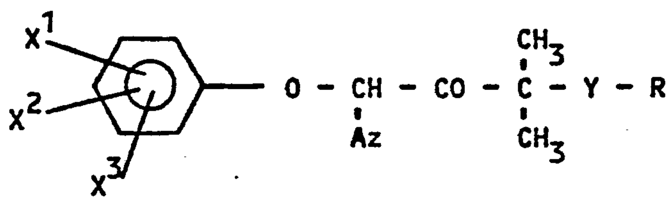

(Ia)

dans laquelle
Az, R, $X^1$, $X^2$, $X^3$ et Y ont les significations indiquées ci-dessus, qu'on réduit éventuellement de la manière habituelle par des procédés connus, et le cas échéant, sur les composés de formule (I) ainsi obtenus, on fixe ensuite par addition un acide ou un sel métallique.

4. Produit fongicide, caractérisé en ce qu'il contient au moins un 1-azolyl-3-méthyl-1-phénoxy-butane-2-one ou -ol substitué en position 3 de formule (I) des revendications 1 et 3.

5. Utilisation des 1-azolyl-3-méthyl-1-phénoxy-butane-2-ones ou -ols substitués en position 3 de formule (I) des revendications 1 et 3 dans la lutte contre les mycètes.